# EUROPEAN PATENT APPLICATION

(11) **EP 4 755 910 A1**
(43) Date of publication of application: **10.06.2026**
(21) Application number: 24848163.2
(22) Date of filing: 26.07.2024
(51) Int. Cl.: C07K 16/24, A61K 39/395, A61P 35/00

(54) **USE OF ANTI-INTERLEUKIN-11 ANTIBODY IN PREPARATION OF DRUG FOR TREATING TUMORS**

(30) Priority: 28.07.2023 CN 202310941870
(71) Applicant: Mabwell (Shanghai) Bioscience Co., Ltd., Shanghai 201210 (CN)
(72) Inventor: ZHANG, Chang, Shanghai 201210 (CN); JIAO, Shasha, Shanghai 201210 (CN); WANG, Rongjuan, Shanghai 201210 (CN); WANG, Shuang, Shanghai 201210 (CN); ZHANG, Jinchao, Shanghai 201210 (CN)
(74) Representative: Heller, Benjamin Henry
(86) International application number: PCT/CN2024/107765
(87) International publication number: WO 2025/026206

(57) **Abstract**

Provided is the use of an anti-human interleukin-11 antibody or an antigen-binding fragment thereof in the preparation of a drug for treating tumors related to IL-11 expression. Experiments demonstrate that the anti-human interleukin-11 antibody or the antigen-binding fragment thereof can bind to IL-11 so as to block the formation of an IL-11/IL-11Rα/GP130 ternary complex and/or block the activation of an STAT3 signaling pathway, thereby achieving an obvious inhibitory effect on tumor growth.

## Description

### CROSS-REFERENCE TO RELATED APPLICATION

This patent application claims priority to Chinese Patent Application No. CN202310941870.8, filed on July 28, 2023, the entire contents of which are incorporated herein by reference.

### TECHNICAL FIELD

The present invention relates to the field of biomedicine and in particular, to use of an anti-interleukin-11 (IL-11) antibody in treatment of tumors.

### BACKGROUND

IL-11 is an important member of the IL-6 family, containing 178 amino acids and having a molecular weight of approximately 19 kDa. IL-11 can be expressed in various cell types, including leukocytes, fibroblasts, and epithelial cells. IL-11 is an important inflammatory factor, and its expression is regulated by a variety of cytokines such as TGF-β, IL-1β, IL-22, IL-17F, IFN-γ, TNF-α, and COX-2. Existing studies have shown that IL-11 plays a significant role in the progression of major diseases such as tumors, inflammatory diseases, acute liver injury, NASH, and fibrosis.

Studies have shown that IL-11 is upregulated in various tumors, including melanoma, breast cancer, colorectal cancer, and non-small cell lung cancer. High expression of IL-11 in non-small cell lung cancer has been found to be closely related to patient prognosis, thus IL-11 is considered a potential biomarker; in breast cancer, upregulated expression of IL-11 can further promote bone metastasis of tumors. Furthermore, IL-11 can bind to the IL-11 receptor alpha (IL-11Rα) and the gp130 receptor, and then activate downstream signaling pathways, including the three classical pathways of JAK/STAT3, Akt, and ERK, in the form of a 2:2:2 6-mer complex of IL-11/IL-11Rα/gp130. Among them, IL-11-induced JAK/STAT3 pathway activation is considered to be closely related to gastrointestinal tumors, and inhibitors of the pathway can be used for tumor treatment; in the process of chronic gastritis turning into gastric cancer, a gradual increase in IL-11 and activation of the signaling pathway were clearly observed. Inhibition of the IL-11 signaling pathway showed a clear inhibitory effect on the growth and metastasis of various tumors, suggesting that the IL-11 signaling pathway plays an important role in maintaining the proliferation, survival and migration of tumor cells.

In recent years, a large number of studies have been conducted on the role of the IL-11 signaling pathway in non-tumor cells such as macrophages, DC cells, CD4+T cells, fibroblasts, etc., enabling people to have a more comprehensive and in-depth understanding of the relationship between IL-11 and tumors. For example, it has been found that IL-11 can suppress the activity of CD4+ positive T cells, thereby achieving immunosuppression; IL-11 induces mDSC differentiation by activating STAT3; tumors, by expressing IL-11, activate IL-11 + fibroblasts, which further chemoattract or activate more IL-11+ fibroblasts to secrete IL-11, forming an suppressive immune microenvironment driven by cancer-associated fibroblasts (CAFs), promoting tumor growth and metastasis; and so on.

The above studies suggest that IL-11 influences the development and progression of tumors in multiple ways. For example, in the tumor microenvironment, tumor cells and non-tumor cells work together through the IL-11 signaling pathway to create an suppressive immune microenvironment suitable for tumor growth. Therefore, inhibitors targeting IL-11 can exert antitumor efficacy through both direct effects on tumor cells and improvement of the suppressive immune microenvironment.

### SUMMARY OF THE INVENTION

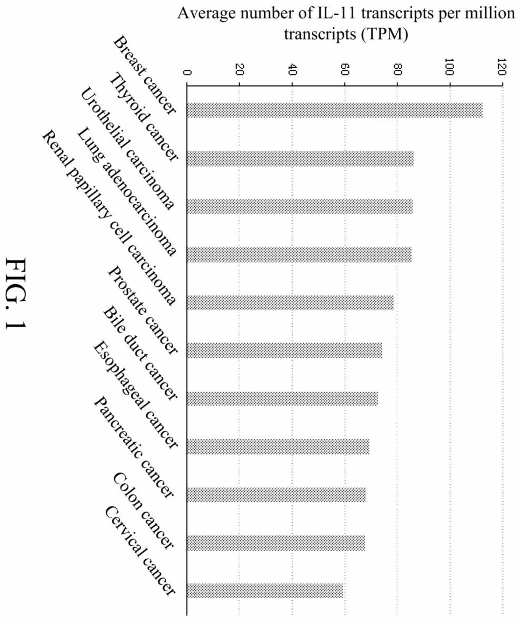

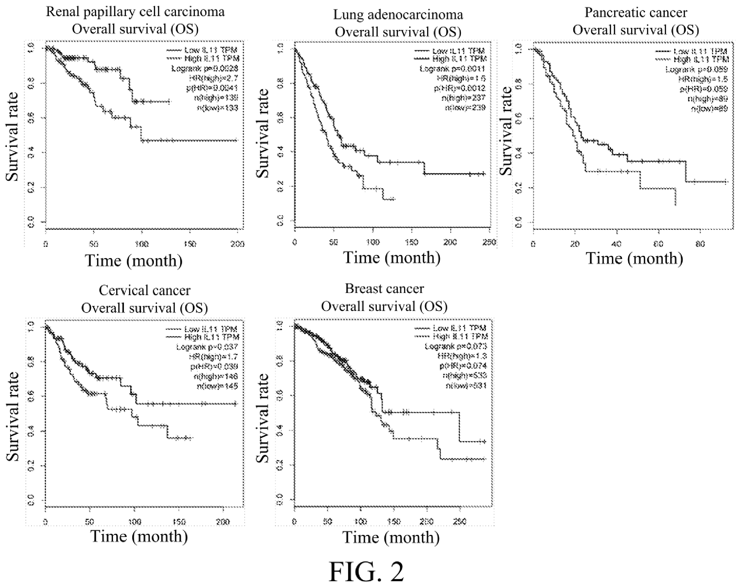

From data from the gepia2 database (http://gepia2.cancer-pku.cn/#general), IL-11 expression is relatively high in breast cancer, thyroid cancer, urothelial carcinoma, lung adenocarcinoma, renal papillary cell carcinoma, prostate cancer, bile duct cancer, esophageal cancer, pancreatic cancer, and colon cancer (FIG. 1). Furthermore, patients with relatively low IL-11 expression in renal papillary cell carcinoma, lung adenocarcinoma, pancreatic cancer, cervical cancer, and breast cancer have a better prognosis (FIG. 2).

Based on the above research results, the inventors attempted to use IL-11 as a tumor treatment target and studied use of anti-IL-11 antibodies in tumor treatment. It was found that antibodies with high affinity for IL-11 had a significant growth inhibitory effect on tumors with high IL-11 expression.

The technical solution of the present invention is as follows.

The present invention provides the use of an anti-IL-11 antibody or antigen-binding fragment thereof in the preparation of a drug for treating tumors associated with IL-11 expression (including overexpression).

In the present invention, the term "IL-11" refers to interleukin-11 derived from mammals. The mammals are, for example, primates (e.g., cynomolgus monkeys, humans) or rodents (e.g., rats, mice).

The "treatment of tumors associated with IL-11 expression (including overexpression)" described in the present invention can means that the effect of inhibiting the proliferation and/or survival of tumor cells or inhibiting tumor growth is achieved. In actual treatment, the anti-IL-11 antibody or antigen-binding fragment thereof can be used alone or in combination with other therapeutic agents or treatments. Accordingly, the present invention includes technical solutions where an anti-IL-11 antibody or antigen-binding fragment thereof is prepared, alone or in combination with other therapeutic agents or treatments, into a drug for treating tumors associated with IL-11 expression (including overexpression).

In the present invention, the term "drug" encompasses those prepared from the anti-IL-11 antibody or antigen-binding fragment thereof that can be administered via any route, such as parenteral, intravenous, intraarterial, intraocular, intraconjunctival, intramuscular, subcutaneous, intradermal, intratumoral injection or infusion, and oral administration. Furthermore, the subject of the drug can be any mammal, preferably a human. The subject can be one who has been diagnosed with a tumor requiring treatment, or who is suspected of having the tumor.

In the present invention, the anti-IL-11 antibody or antigen-binding fragment thereof comprises a heavy chain variable region (VH) and/or a light chain variable region (VL). The heavy chain variable region (VH) may comprise heavy chain CDR1, heavy chain CDR2, and heavy chain CDR3 (H-CDR1, H-CDR2, and H-CDR3) derived from a heavy chain variable region having the amino acid sequence shown in SEQ ID NO. 1, SEQ ID NO. 3, SEQ ID NO. 5, SEQ ID NO. 7, SEQ ID NO. 9, or SEQ ID NO. 11. The light chain variable region (VL) may comprise light chain CDR1, light chain CDR2, and light chain CDR3 (L-CDR1, L-CDR2, and L-CDR3) derived from a light chain variable region having the amino acid sequence shown in SEQ ID NO. 2, SEQ ID NO. 4, SEQ ID NO. 6, SEQ ID NO. 8, SEQ ID NO. 10, or SEQ ID NO. 12.

Each of the amino acid sequences described above respectively serves as the amino acid sequence of a heavy chain variable region or a light chain variable region of the anti-IL-11 antibody provided in the present invention. By using systems for defining complementarity-determining regions (CDRs) in an antibody heavy or light chain variable region (e.g., Chothia, Kabat, IMGT, Contact, etc.) that are well-known in the art, those skilled in the art can easily determine the heavy chain CDRs and light chain CDRs contained therein. In a specific embodiment of the present invention, the Kabat system can be used to delineate the CDRs in the variable region sequence.

Preferably, the anti-IL-11 antibody or antigen-binding fragment thereof comprises a heavy chain variable region (VH) and a light chain variable region (VL), and the heavy chain variable region and light chain variable region comprise heavy chain CDR1, heavy chain CDR2, and heavy chain CDR3 (H-CDR1, H-CDR2, and H-CDR3) and light chain CDR1, light chain CDR2, and light chain CDR3 (L-CDR1, L-CDR2, and L-CDR3) from a heavy chain variable region and light chain variable region as shown in the following amino acid sequence pairings:
(1) SEQ ID NO. 1 + SEQ ID NO. 2;
(2) SEQ ID NO. 3 + SEQ ID NO. 4;
(3) SEQ ID NO. 5 + SEQ ID NO. 6;
(4) SEQ ID NO. 7 + SEQ ID NO. 8;
(5) SEQ ID NO. 9 + SEQ ID NO. 10; or
(6) SEQ ID NO. 11 + SEQ ID NO. 12.

As mentioned above, the Kabat system can be used to delineate the CDRs in each variable region sequence of the above amino acid sequence pairings.

In a specific embodiment of the present invention, the heavy chain variable region and light chain variable region in the antibody or antigen-binding fragment thereof comprise a combination (H-CDR1, H-CDR2, and H-CDR3; and L-CDR1, L-CDR2, and L-CDR3) of heavy chain CDRs and light chain CDRs selected from:
(1) H-CDR1, H-CDR2, and H-CDR3 comprising the amino acid sequences shown in SEQ ID NO. 26, SEQ ID NO. 27, and SEQ ID NO. 28, respectively; and L-CDR1, L-CDR2, and L-CDR3 comprising the amino acid sequences shown in SEQ ID NO. 29, SEQ ID NO. 30, and SEQ ID NO. 31, respectively;
(2) H-CDR1, H-CDR2, and H-CDR3 comprising the amino acid sequences shown in SEQ ID NO. 26, SEQ ID NO. 27, and SEQ ID NO. 32, respectively; and L-CDR1, L-CDR2, and L-CDR3 comprising the amino acid sequences shown in SEQ ID NO. 29, SEQ ID NO. 33, and SEQ ID NO. 31, respectively;
(3) H-CDR1, H-CDR2, and H-CDR3 comprising the amino acid sequences shown in SEQ ID NO. 34, SEQ ID NO. 35, and SEQ ID NO. 36, respectively; and L-CDR1, L-CDR2, and L-CDR3 comprising the amino acid sequences shown in SEQ ID NO. 37, SEQ ID NO. 38, and SEQ ID NO. 39, respectively;
(4) H-CDR1, H-CDR2, and H-CDR3 comprising the amino acid sequences shown in SEQ ID NO. 26, SEQ ID NO. 27, and SEQ ID NO. 40, respectively; and L-CDR1, L-CDR2, and L-CDR3 comprising the amino acid sequences shown in SEQ ID NO. 29, SEQ ID NO. 33, and SEQ ID NO. 31, respectively;
(5) H-CDR1, H-CDR2, and H-CDR3 comprising the amino acid sequences shown in SEQ ID NO. 41, SEQ ID NO. 42, and SEQ ID NO. 36, respectively; and L-CDR1, L-CDR2, and L-CDR3 comprising the amino acid sequences shown in SEQ ID NO. 43, SEQ ID NO. 38, and SEQ ID NO. 39, respectively; or
(6) H-CDR1, H-CDR2, and H-CDR3 comprising the amino acid sequences shown in SEQ ID NO. 44, SEQ ID NO. 42, and SEQ ID NO. 36, respectively; and L-CDR1, L-CDR2, and L-CDR3 comprising the amino acid sequences shown in SEQ ID NO. 37, SEQ ID NO. 38, and SEQ ID NO. 39, respectively.

Preferably, the heavy chain variable region (VH) and light chain variable region (VL) in the anti-IL-11 antibody or antigen-binding fragment thereof respectively comprise a combination of amino acid sequences selected from:
(1) the amino acid sequence shown in SEQ ID NO. 1, or an amino acid sequence having at least 75% identity with said amino acid sequence; and the amino acid sequence shown in SEQ ID NO. 2, or an amino acid sequence having at least 75% identity with said amino acid sequence;
(2) the amino acid sequence shown in SEQ ID NO. 3, or an amino acid sequence having at least 75% identity with said amino acid sequence; and the amino acid sequence shown in SEQ ID NO. 4, or an amino acid sequence having at least 75% identity with said amino acid sequence;
(3) the amino acid sequence shown in SEQ ID NO. 5, or an amino acid sequence having at least 75% identity with said amino acid sequence; and the amino acid sequence shown in SEQ ID NO. 6, or an amino acid sequence having at least 75% identity with said amino acid sequence;
(4) the amino acid sequence shown in SEQ ID NO. 7, or an amino acid sequence having at least 75% identity with said amino acid sequence; and the amino acid sequence shown in SEQ ID NO. 8, or an amino acid sequence having at least 75% identity with said amino acid sequence;
(5) the amino acid sequence shown in SEQ ID NO. 9, or an amino acid sequence having at least 75% identity with said amino acid sequence; and the amino acid sequence shown in SEQ ID NO. 10, or an amino acid sequence having at least 75% identity with said amino acid sequence; or
(6) the amino acid sequence shown in SEQ ID NO. 11, or an amino acid sequence having at least 75% identity with said amino acid sequence; and the amino acid sequence shown in SEQ ID NO. 12, or an amino acid sequence having at least 75% identity with said amino acid sequence.

In the present invention, at most 25% difference in amino acid sequence resulting from the "at least 75% identity" may exist in any framework region within the heavy chain variable region or light chain variable region, or in any domain or sequence outside the heavy chain variable region and light chain variable region of the antibody or fragment thereof of the present invention. The difference can be caused by the deletion, addition, or substitution of amino acids at any position, wherein the substitution can be a conservative substitution or a non-conservative substitution. The "at least 75% identity" refers to any percentage of identity between at least 75% and 100% identity, such as 75%, 80%, 85%, 90%, or even 91%, 92%, 93%, 94%, 95%, 96%, 97%, 98%, 99%, or even 100% identity.

The anti-IL-11 antibody or antigen-binding fragment thereof comprising the above amino acid sequence can be an antibody or antigen-binding thereof of any structural configuration. For example, the antibody itself can be a monoclonal antibody, a bispecific or multispecific antibody, or an antibody with a structure such as scFv, BsFv, dsFv, (dsFv)2, Fab, Fab', F(ab')2 or Fv; or any fragment in any form.

More preferably, the present invention provides the use of the anti-IL-11 antibody or antigen-binding fragment thereof in the preparation of a drug for treating tumors associated with IL-11 expression (including overexpression), wherein the anti-IL-11 antibody is a humanized antibody. Preferably, the anti-IL-11 antibody comprises an IgG heavy chain constant region and/or a κ or λ light chain constant region. In a specific embodiment of the present invention, the anti-IL-11 antibody comprises an IgG1 heavy chain constant region and a κ light chain constant region. More specifically, the anti-IL-11 antibody is an IgG1 or κ monoclonal antibody.

In a specific embodiment of the present invention, the anti-IL-11 antibody or antigen-binding fragment thereof comprises a heavy chain (HC) and light chain (LC) respectively comprising a combination of amino acid sequences selected from:
(1) the amino acid sequence shown in SEQ ID NO. 13, or an amino acid sequence having at least 75% identity with said amino acid sequence; and the amino acid sequence shown in SEQ ID NO. 14, or an amino acid sequence having at least 75% identity with said amino acid sequence;
(2) the amino acid sequence shown in SEQ ID NO. 15, or an amino acid sequence having at least 75% identity with said amino acid sequence; and the amino acid sequence shown in SEQ ID NO. 16, or an amino acid sequence having at least 75% identity with said amino acid sequence;
(3) the amino acid sequence shown in SEQ ID NO. 17, or an amino acid sequence having at least 75% identity with said amino acid sequence; and the amino acid sequence shown in SEQ ID NO. 18, or an amino acid sequence having at least 75% identity with said amino acid sequence;
(4) the amino acid sequence shown in SEQ ID NO. 19, or an amino acid sequence having at least 75% identity with said amino acid sequence; and the amino acid sequence shown in SEQ ID NO. 20, or an amino acid sequence having at least 75% identity with said amino acid sequence;
(5) the amino acid sequence shown in SEQ ID NO. 21, or an amino acid sequence having at least 75% identity with said amino acid sequence; and the amino acid sequence shown in SEQ ID NO. 22, or an amino acid sequence having at least 75% identity with said amino acid sequence; or
(6) the amino acid sequence shown in SEQ ID NO. 23, or an amino acid sequence having at least 75% identity with said amino acid sequence; and the amino acid sequence shown in SEQ ID NO. 24, or an amino acid sequence having at least 75% identity with said amino acid sequence.

In a specific embodiment of the present invention, the heavy chain constant region of the anti-IL-11 antibody or antigen-binding fragment thereof can be replaced with a heavy chain constant region shown in SEQ ID NO. 25.

Preferably, the tumor is a tumor associated with IL-11 expression (including overexpression), such as non-small cell lung cancer (lung adenocarcinoma, lung squamous cell carcinoma), pancreatic cancer, or cervical cancer.

In the use provided by the present invention, the drug is used for treating a mammal, preferably a human. The mammal can be one who has been diagnosed with a tumor requiring treatment, or who is suspected of having the tumor.

In another aspect, the present invention provides a method for treating a tumor, comprising administering to a subject in need thereof an anti-IL-11 antibody or antigen-binding fragment thereof. The tumor is a tumor associated with IL-11 expression (including overexpression), such as non-small cell lung cancer (lung adenocarcinoma, lung squamous cell carcinoma), pancreatic cancer, or cervical cancer. The anti-IL-11 antibody or antigen-binding fragment thereof is as defined above.

In the method, the route of administration can be parenteral, intravenous, intraarterial, intraocular, intraconjunctival, intramuscular, subcutaneous, intradermal, intratumoral injection or infusion, and oral administration. Furthermore, the subject can be any mammal, preferably a human. The subject can be one who has been diagnosed with a tumor requiring treatment, or who is suspected of having the tumor.

Preferably, in the method, the subject is administered with a therapeutically effective amount of the anti-IL-11 antibody or antigen-binding fragment thereof. In the present invention, the term "therapeutically effective amount" refers to an amount that is capable of demonstrating a therapeutic benefit in a subject. The administered actual dosage or administration regimen may depend on factors such as the nature and severity of the tumor.

The method for treating a tumor provided by the present invention encompasses administration of the anti-IL-11 antibody or antigen-binding fragment thereof alone or in combination with other therapeutic agents or treatments. In the present invention, the term "combination" administration refers to administration of the anti-IL-11 antibody or antigen-binding fragment thereof concurrently with or sequentially at certain time intervals to other therapeutic agents or treatments; the time intervals can be any time intervals.

In contrast to the prior art, the present invention proposes a new approach to treat tumors by using IL-11 as a tumor treatment target. Without being bound by any theory, in the present invention, a specific anti-interleukin-11 antibody is used as a therapeutic agent. By binding to IL-11 with high affinity, it blocks the formation of the IL-11/IL-11Rα/GP130 ternary complex and/ or blocks the activation of the STAT3 signaling pathway, thereby achieving a significant inhibitory effect on tumor growth.

### DESCRIPTION OF THE DRAWINGS

Hereinafter, embodiments of the present invention are described in detail with reference to the accompanying drawings, in which:
FIG. 1 shows the expression of IL-11 at the transcript level in tumor tissue.
FIG. 2 shows the relationship between IL-11 and prognosis in patients with tumors.
FIG. 3 shows the tumor growth curves (volume) over time after subcutaneous transplantation of IL-11Rα-knockout MC38 cells and wild-type MC38 cells in mice.
FIG. 4 shows the tumor growth curves (volume) over time after subcutaneous implantation of MC38 cells in wild-type mice and IL-11Rα-knockout mice.
FIG. 5 shows the tumor growth following administration of different antibodies in a mouse model of human lung adenocarcinoma; wherein, 5A: tumor growth curves (volume); 5B: tumor weight.
FIG. 6 shows the tumor growth following administration of different antibodies in a PDX mouse model of human lung adenocarcinoma; wherein, 6A: tumor growth curves (volume); 6B: tumor weight.
FIG. 7 shows the tumor growth curves (volume) following administration of different antibodies in a PDX mouse model of human pancreatic cancer.
FIG. 8 shows the tumor growth following administration of different antibodies in a PDX mouse model of human cervical cancer; wherein, 8A: tumor growth curves (volume); 8B: tumor weight.
FIG. 9 shows the tumor growth curves (volume) following administration of different antibodies in a PDX mouse model of human lung squamous cell carcinoma.

### BEST MODE FOR CARRYING OUT THE INVENTION

The present invention will be described below with reference to specific examples. Those skilled in the art will understand that these examples are for illustrative purposes only and do not limit the scope of the invention in any way.

Unless otherwise specified, all experimental methods in the following examples are conventional methods. Unless otherwise specified, all raw materials and reagents used in the following examples are commercially available products.

The anti-IL-11 antibodies hz8C8m7, hz8C8m13, and hz18A10m19 were used in the following examples. These three antibodies are humanized monoclonal antibodies obtained by humanizing murine antibodies, each comprising two heavy chains and two light chains. Experiments have shown that all three antibodies can bind to human IL-11 with high affinity and can block the formation of the IL-11/IL-11Rα/gp130 ternary complex; see Chinese Invention Patent CN202310044074.4.

The amino acid sequences of the antibodies employed in the present invention are set forth below, wherein the italicized sequences represent heavy or light chain variable regions; within the variable regions, the underlined and bolded sequences sequentially represent CDR1 to CDR3 (as delineated by the Kabat system); and the sequences that are bolded only represent heavy or light chain constant regions:
1. hz8C8m7:
   > Heavy chain 
EVQLVESGGGLVQPGGSLRLSCAASGFTFSSFGMHWVRQAPGKGLEWVAYITPGSNTIY
YADTVKGRFTISRDNAKNSLYLQMNSLRAEDTAVYYCAREYYSYDEGFAYWGQGTLVTVSSA
STKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPR
EPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK
      H-CDR1: SEQ ID NO. 26
      H-CDR2: SEQ ID NO. 27
      H-CDR3: SEQ ID NO. 28
      VH: SEQ ID NO. 1
      Heavy chain full length: SEQ ID NO. 13
   >Light chain 
EIVLTQSPATLSLSPGERATLSCSVSDLIPSQYLHWYQQKPGQAPRLLIYRTSNLASGIPAR
FSGSGSGTDFTLTISSLEPEDFAVYYCQQGVDVPFTFGQGTKLEIKRTVAAPSVFIFPPSDE
QLKSGTASVVCLLNNFYPREAKVQWKVDNALQSGNSQESVTEQDSKDSTYSLSSTL
TLSKADYEKHKVYACEVTHQGLSSPVTKSFNRGEC
      L-CDR1: SEQ ID NO. 29
      L-CDR2: SEQ ID NO. 30
      L-CDR3: SEQ ID NO. 31
      VL: SEQ ID NO. 2
      Light chain full length: SEQ ID NO. 14
2. hz8C8m13
   > Heavy chain 
EVQLVESGGGLVQPGGSLRLSCAASGFTFSSFGMHWVRQAPGKGLEWVAYITPGSNTIY
YADTVKGRFTISRDNAKNSLYLQMNSLRAEDTAVYYCAREYYSYDEGFASWGQGTLVTVSSA
STKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPR
EPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK
      H-CDR1: SEQ ID NO. 26
      H-CDR2: SEQ ID NO. 27
      H-CDR3: SEQ ID NO. 32
      VH: SEQ ID NO. 3
      Heavy chain full length: SEQ ID NO. 15
   > Light chain 
EIVLTQSPATLSLSPGERATLSCSVSDLIPSQYLHWYQQKPGQAPRLLIYDASNLASGIPAR
FSGSGSGTDFTLTISSLEPEDFAVYYCQQGVDVPFTFGQGTKLEIKRTVAAPSVFIFPPSDE
QLKSGTASVVCLLNNFYPREAKVQWKVDNALQSGNSQESVTEQDSKDSTYSLSSTL
TLSKADYEKHKVYACEVTHQGLSSPVTKSFNRGEC
      L-CDR1: SEQ ID NO. 29
      L-CDR2: SEQ ID NO. 33
      L-CDR3: SEQ ID NO. 31
      VL: SEQ ID NO. 4
      Light chain full length: SEQ ID NO. 16
3. hz18A10m19
   > Heavy chain 
EVQLVQSGAEVKKPGASVKVSCKASGYTFTHYNMDWVRQAPGQGLEWMGTIYPGNADT
SYNQKFKGRVTMTRDTSTSTVYMELSSLRSEDTAVYYCARGDLYALDYWGQGTLVTVSSAST
KGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGS
FFLYSKLTVDKSRWQQGNVFSCSVMHEALHNHYTQKSLSLSPGK
      H-CDR1: SEQ ID NO. 34
      H-CDR2: SEQ ID NO. 35
      H-CDR3: SEQ ID NO. 36
      VH: SEQ ID NO. 5
      Heavy chain full length: SEQ ID NO. 17
   > Light chain 
DIQMTQSPSSLSASVGDRVTITCKASQSVYRDVAWYQQKPGKAPKLLIYSASNRYTGVPSR
FSGSGSGTDFTFTISSLQPEDIATYYCQHDYSSPYTFGQGTKLEIKRTVAAPSVFIFPPSDEQ
LKSGTASVVCLLNNFYPREAKVQWKVDNALQSGNSQESVTEQDSKDSTYSLSSTLT
LSKADYEKHKVYACEVTHQGLSSPVTKSFNRGEC
      L-CDR1: SEQ ID NO. 37
      L-CDR2: SEQ ID NO. 38
      L-CDR3: SEQ ID NO. 39
      VL: SEQ ID NO. 6
      Light chain full length: SEQ ID NO. 18
4. hz8C8m6
   > Heavy chain 
EVQLVESGGGLVQPGGSLRLSCAASGFTFSSFGMHWVRQAPGKGLEWVAYITPGSNTIY
YADTVKGRFTISRDNAKNSLYLQMNSLRAEDTAVYYCAREYSSYDEGFAYWGQGTLVTVSSA
STKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPR
EPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK
      H-CDR1: SEQ ID NO. 26
      H-CDR2: SEQ ID NO. 27
      H-CDR3: SEQ ID NO. 40
      VH: SEQ ID NO. 7
      Heavy chain full length: SEQ ID NO.19
   > Light chain 
EIVLTQSPATLSLSPGERATLSCSVSDLIPSQYLHWYQQKPGQAPRLLIYDASNLASGIPAR
FSGSGSGTDFTLTISSLEPEDFAVYYCQQGVDVPFTFGQGTKLEIKRTVAAPSVFIFPPSDE
QLKSGTASVVCLLNNFYPREAKVQWKVDNALQSGNSQESVTEQDSKDSTYSLSSTL
TLSKADYEKHKVYACEVTHQGLSSPVTKSFNRGEC
      L-CDR1: SEQ ID NO. 29
      L-CDR2: SEQ ID NO. 33
      L-CDR3: SEQ ID NO. 31
      VL: SEQ ID NO. 8
      Light chain full length: SEQ ID NO. 20
5. hz18A10
   > Heavy chain 
EVQLVQSGAEVKKPGASVKVSCKASGYTFTTYNMDWVRQAPGQGLEWMGTIYPGNGDT
SYNQKFKGRVTMTRDTSTSTVYMELSSLRSEDTAVYYCARGDLYALDYWGQGTLVTVSSAST
KGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTK
 
PREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGS
FFLYSKLTVDKSRWQQGNVFSCSVMHEALHNHYTQKSLSLSPGK
      H-CDR1: SEQ ID NO. 41
      H-CDR2: SEQ ID NO. 42
      H-CDR3: SEQ ID NO. 36
      VH: SEQ ID NO. 9
      Heavy chain full length: SEQ ID NO. 21
   > Light chain 
DIQMTQSPSSLSASVGDRVTITCKASQSVSNDVAWYQQKPGKAPKLLIYSASNRYTGVPSR
FSGSGSGTDFTFTISSLQPEDIATYYCQHDYSSPYTFGQGTKLEIKRTVAAPSVFIFPPSDEQ
LKSGTASVVCLLNNFYPREAKVQWKVDNALQSGNSQESVTEQDSKDSTYSLSSTLT
LSKADYEKHKVYACEVTHQGLSSPVTKSFNRGEC
      L-CDR1: SEQ ID NO. 43
      L-CDR2: SEQ ID NO. 38
      L-CDR3: SEQ ID NO. 39
      VL: SEQ ID NO. 10
      Light chain full length: SEQ ID NO. 22
6. hz18A10m6
   > Heavy chain 
EVQLVQSGAEVKKPGASVKVSCKASGYTFNHYDMDWVRQAPGQGLEWMGTIYPGNGD
TSYNQKFKGRVTMTRDTSTSTVYMELSSLRSEDTAVYYCARGDLYALDYWGQGTLVTVSSAS
TKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPRE
PQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK
      H-CDR1: SEQ ID NO. 44
      H-CDR2: SEQ ID NO. 42
      H-CDR3: SEQ ID NO. 36
      VH: SEQ ID NO. 11
      Heavy chain full length: SEQ ID NO. 23
   > Light chain 
DIQMTQSPSSLSASVGDRVTITCKASQSVYRDVAWYQQKPGKAPKLLIYSASNRYTGVPSR
FSGSGSGTDFTFTISSLQPEDIATYYCQHDYSSPYTFGQGTKLEIKRTVAAPSVFIFPPSDEQ
LKSGTASVVCLLNNFYPREAKVQWKVDNALQSGNSQESVTEQDSKDSTYSLSSTLT
LSKADYEKHKVYACEVTHQGLSSPVTKSFNRGEC
      L-CDR1: SEQ ID NO. 37
      L-CDR2: SEQ ID NO. 38
      L-CDR3: SEQ ID NO. 39
      VL: SEQ ID NO. 12
      Light chain full length: SEQ ID NO. 24

The heavy chain constant regions of the above-mentioned antibodies may each be replaced with a heavy chain constant region as set forth below (SEQ ID NO. 25):ASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK

### Example 1: Effect of blocking the IL-11 signaling pathway on tumor growth

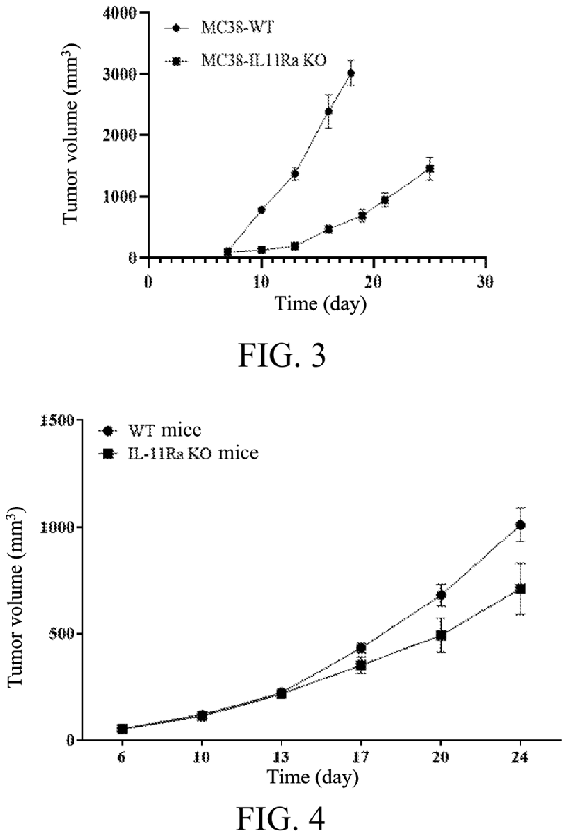

IL-11Rα-knockout mouse colorectal cancer cells, MC38 cells, were obtained (purchased from KYinno Biotechnology Co., Ltd., KC-2286). The cells were named "MC38-IL-11Rα KO cells"; IL-11Rα was knocked out to block the IL-11 signaling pathway on the cells. MC38-IL-11Rα KO cells and wild-type MC38 cells (named "MC38-WT cells") were transplanted subcutaneously into C57BL/6 mice at the same dose, and tumor growth was observed over time. The results (FIG. 3) show that, compared to MC38-WT cells, the tumor growth rate in mice transplanted with MC38-IL-11Rα KO cells is significantly slower.

C57BL/mice with IL-11Rα knockout (purchased from Shanghai Model Organisms Center, Inc., catalog no. NM-KO-190453) were obtained and named IL-11Rα KO mice. MC38-WT cells were inoculated at the same dose into IL-11Rα KO mice and wild-type C57BL/ mice (named "WT mice"), respectively, and tumor growth was observed over time. The results (FIG. 4) show that, compared to WT mice, the tumor growth rate in IL-11Rα KO mice is significantly slower.

From the above results, it can be seen that the IL-11 signaling pathway acts on both tumor cells and non-tumor cells within the host immune microenvironment, thereby promoting the growth and development of tumors from multiple perspectives. Therefore, neutralizing antibodies that block IL-11 may have potential benefits in the treatment of tumors.

### Example 2: Antitumor efficacy of anti-IL-11 antibody

### (1) Evaluation of the antitumor efficacy of anti-IL-11 antibody in a mouse model bearing human lung adenocarcinoma cells

Six-week-old female Balb/C nude mice were subcutaneously inoculated with 3×10⁶ human lung adenocarcinoma A549 cells. When the tumors grew to about 60 mm³, they were randomly divided into groups of 8 mice each. The grouping and administration dosage and frequency are shown in Table 1. During administration, tumor volume and mouse body weight were measured. When the mouse body weight loss exceeded 15%, or when the tumor volume of a single animal exceeded 3000 mm³ or the average tumor volume of a group of animals exceeded 2000 mm³, the experiment was terminated for the relevant mice, which were then euthanized.

**Table 1. Animal grouping and administration of antibodies in a mouse model bearing human lung adenocarcinoma cells**

| Group (n=8) | Drug | Administration dosage (mg/kg) | Administration mode | Administration frequency |
|---|---|---|---|---|
| 1 | hz8C8m7 | 0.4 | i.p. | Biw×9 |
| 2 | hz8C8m7 | 2 | i.p. | Biw×9 |
| 3 | hz8C8m13 | 10 | i.p. | Biw×9 |
| 4 | hz18A10m19 | 10 | i.p. | Biw×9 |
| 5 | NC-hIgG1 | 10 | i.p. | Biw×9 |

At the end of the experiment, the tumors of the euthanized mice were removed and weighed, and the tumor growth volume inhibition rate (TGI_{TV} (%)) and tumor weight inhibition rate (TGI_{TW} (%)) were calculated respectively:
The formula for calculating tumor volume (TV) is: Volume (TV) = 0.5 × major diameter × minor diameter². The T/C value was calculated based on tumor volume (TV) and relative tumor volume (RTV), where RTV is the ratio of tumor volume after administration to tumor volume before administration, and T/C = mean RTV of treatment group/mean RTV of control group. Tumor growth volume inhibition rate (TGI_{TV} (%)) = (1 - T/C) × 100%.

The T/C value was calculated based on the tumor weight (TW) of the treatment group and the tumor weight (TW) of the control group. T/C = mean tumor weight (TW) of treatment group/mean tumor weight (TW) of control group. Tumor weight inhibition rate TGI_{TW} (%) = (1 - T/C) × 100%.

Statistical difference analysis was performed using T-test.

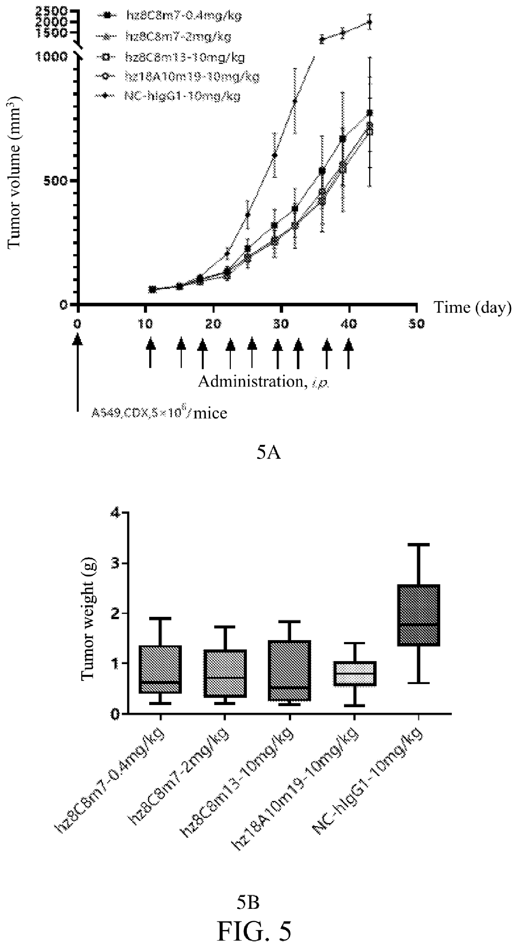

The results (FIG. 5, Table 2) show that, compared with the control mice administered with the isotype control antibody, all of the anti-IL-11 antibodies could clearly inhibit tumor growth, with statistical significance.

**Table 2. Tumor growth inhibition rate following administration of antibodies in a mouse model bearing human lung adenocarcinoma cells**

| Group | Tumor volume | | Tumor weight | |
|---|---|---|---|---|
| | Tumor volume inhibition rate | P-value | Tumor weight inhibition rate | P-value |
| hz8C8m7-0.4mg/kg | 63% | 0.0110 | 43% | 0.0323 |
| hz8C8m7-2mg/kg | 64% | 0.0072 | 42% | 0.0296 |
| hz8C8m13-10mg/kg | 68% | 0.0059 | 41% | 0.0256 |
| hz18A10m19-10mg/kg | 64% | 0.0074 | 42% | 0.0297 |
| NC-hIgG1-10mg/kg | / | / | / | / |

### (2) Evaluation of the antitumor efficacy of anti-IL-11 antibody in a PDX mouse model bearing different types of tumors

Six-week-old female Balb/C nude mice were subcutaneously inoculated with tumor fragments of human lung adenocarcinoma (passage P7), human lung squamous cell carcinoma (passage P10), human cervical cancer (passage P6), and human pancreatic cancer (passage P9) (all tumor fragments were provided by Yicon (Beijing) BioMedical Technology Inc.), with an inoculation volume of 2 mm×2 mm×2 mm/mouse. When the tumors grew to 70 mm³-100 mm³, they were randomly divided into two groups. The grouping and administration dosage and frequency are shown in Table 3. During administration, tumor volume and mouse body weight were measured. When the mouse body weight loss exceeded 15%, or when the tumor volume of a single animal exceeded 3000 mm³ or the average tumor volume of a group of animals exceeded 2000 mm³, the experiment was terminated for the relevant mice, which were then euthanized.

**Table 3 Animal grouping and administration of antibodies in a PDX mouse model bearing different types of tumors**

| PDX tumor type | Group | Drug | Administration dosage (mg/kg) | Administration mode | Administration frequency |
|---|---|---|---|---|---|
| Lung adenocarcinoma YK-Luc-023 | 1 (n=8) | hz8C8m7 | 10 | i.p. | Biw×9 |
| | 2 (n=7) | NC-hIgG1 | 10 | i.p. | Biw×9 |
| Lung squamous cell carcinoma YK-Luc-008 | 1 (n=6) | hz8C8m7 | 10 | i.p. | Biw×5 |
| | 2 (n=6) | NC-hIgG1 | 10 | i.p. | Biw×5 |
| Cervical cancer YK-CSEC-001 | 1 (n=8) | hz8C8m7 | 10 | i.p. | Biw×9 |
| | 2 (n=8) | NC-hIgG1 | 10 | i.p. | Biw×9 |
| Pancreatic cancer YK-PAAD-008 | 1 (n=8) | hz8C8m7 | 10 | i.p. | Biw×4 |
| | 2 (n=8) | NC-hIgG1 | 10 | i.p. | Biw×4 |

At the end of the experiment, the tumors of the euthanized mice were removed and weighed, and the tumor growth volume inhibition rate (TGI_{TV} (%)) and tumor weight inhibition rate ((TGI_{TW} (%)) were calculated respectively in the same manner as described above.

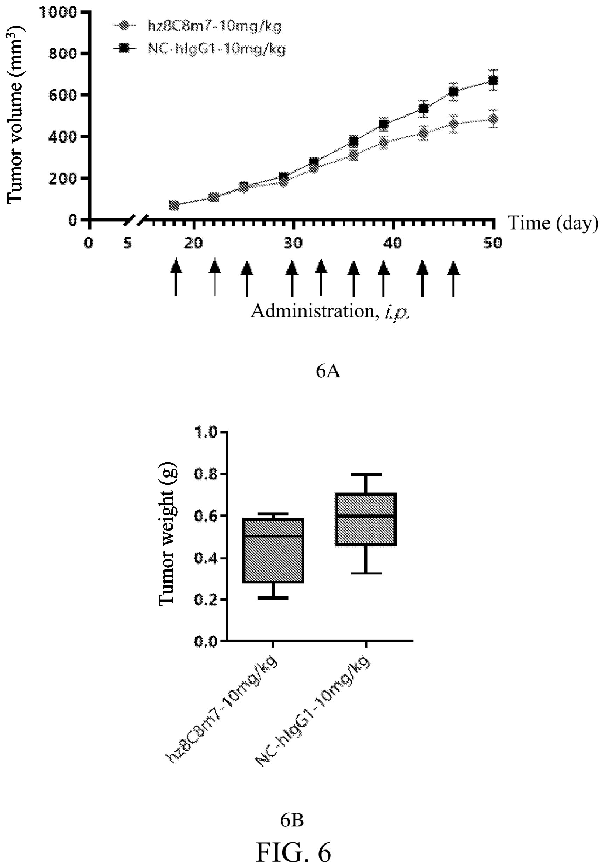

In a PDX mouse model bearing human lung adenocarcinoma, the results (FIG. 6) show that, compared with the control mice administered with the isotype control antibody, the anti-IL-11 antibody hz8C8m7 could clearly inhibit tumor growth, with TGI_{TV} = 28% (p<0.05), and TGI_{TW} = 23%.

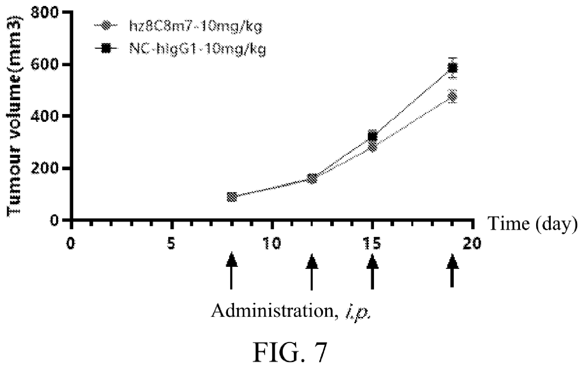

In a PDX mouse model bearing human pancreatic cancer, the results (FIG. 7) show that, compared with the control mice administered with the isotype control antibody, the anti-IL-11 antibody hz8C8m7 could clearly inhibit tumor growth, with TGI_{TV} = 18% (p<0.05).

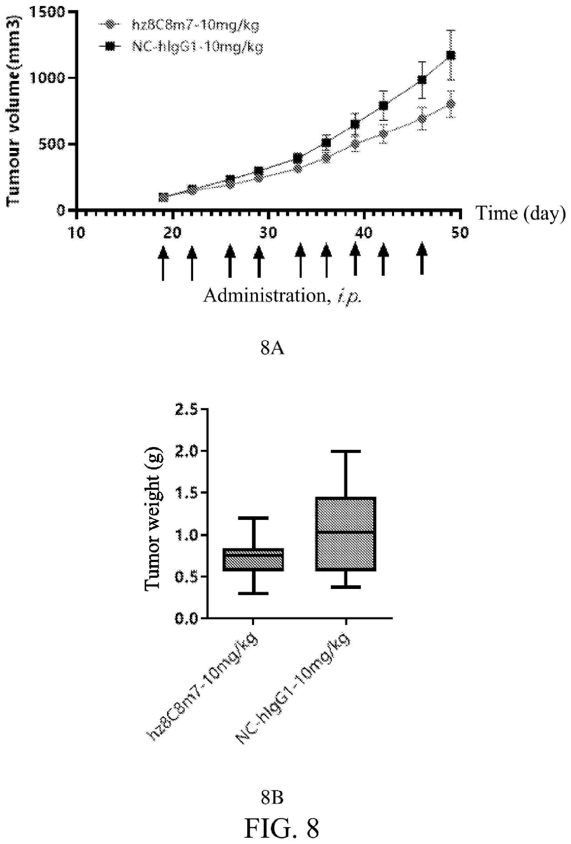

In a PDX mouse model bearing human cervical cancer, the results (FIG. 8) show that, compared with the control mice administered with the isotype control antibody, the anti-IL-11 antibody hz8C8m7 could clearly inhibit tumor growth, with TGI_{TV} = 31%, and TGI_{TW} = 31%.

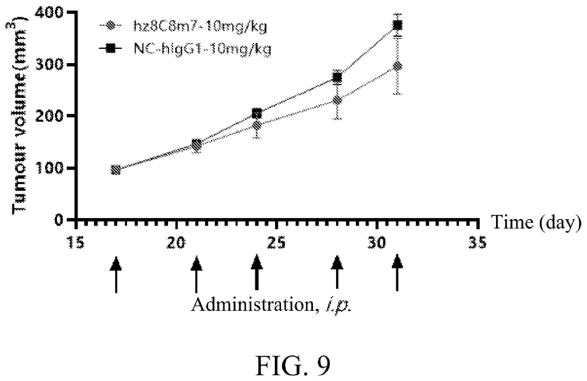

In a PDX mouse model bearing human lung squamous cell carcinoma, the results (FIG. 9) show that, compared with the control mice administered with the isotype control antibody, the anti-IL-11 antibody hz8C8m7 could clearly inhibit tumor growth, with TGI_{TV} = 23%.

The foregoing description of the specific embodiments of the present invention is not intended to limit the invention. Various modifications and variations that can be made by those skilled in the art based on the present invention shall fall within the scope of the appended claims without departing from the spirit of the invention.

## Claims

1. Use of an anti-IL-11 antibody or antigen-binding fragment thereof in the preparation of a drug for treating tumors associated with IL-11 expression.

2. The use according to claim 1, wherein the drug for treating tumors associated with IL-11 expression is capable of inhibiting the proliferation and/or survival of tumor cells, or inhibiting tumor growth.

3. The use according to claim 1 or 2, wherein the anti-IL-11 antibody or antigen-binding fragment thereof comprises a heavy chain variable region (VH) and light chain variable region (VL) comprising a combination (H-CDR1, H-CDR2, and H-CDR3; and L-CDR1, L-CDR2, and L-CDR3) of heavy chain CDRs and light chain CDRs selected from:
(1) H-CDR1, H-CDR2, and H-CDR3 comprising the amino acid sequences shown in SEQ ID NO. 26, SEQ ID NO. 27, and SEQ ID NO. 28, respectively; and L-CDR1, L-CDR2, and L-CDR3 comprising the amino acid sequences shown in SEQ ID NO. 29, SEQ ID NO. 30, and SEQ ID NO. 31, respectively;
(2) H-CDR1, H-CDR2, and H-CDR3 comprising the amino acid sequences shown in SEQ ID NO. 26, SEQ ID NO. 27, and SEQ ID NO. 32, respectively; and L-CDR1, L-CDR2, and L-CDR3 comprising the amino acid sequences shown in SEQ ID NO. 29, SEQ ID NO. 33, and SEQ ID NO. 31, respectively;
(3) H-CDR1, H-CDR2, and H-CDR3 comprising the amino acid sequences shown in SEQ ID NO. 34, SEQ ID NO. 35, and SEQ ID NO. 36, respectively; and L-CDR1, L-CDR2, and L-CDR3 comprising the amino acid sequences shown in SEQ ID NO. 37, SEQ ID NO. 38, and SEQ ID NO. 39, respectively;
(4) H-CDR1, H-CDR2, and H-CDR3 comprising the amino acid sequences shown in SEQ ID NO. 26, SEQ ID NO. 27, and SEQ ID NO. 40, respectively; and L-CDR1, L-CDR2, and L-CDR3 comprising the amino acid sequences shown in SEQ ID NO. 29, SEQ ID NO. 33, and SEQ ID NO. 31, respectively;
(5) H-CDR1, H-CDR2, and H-CDR3 comprising the amino acid sequences shown in SEQ ID NO. 41, SEQ ID NO. 42, and SEQ ID NO. 36, respectively; and L-CDR1, L-CDR2, and L-CDR3 comprising the amino acid sequences shown in SEQ ID NO. 43, SEQ ID NO. 38, and SEQ ID NO. 39, respectively; or
(6) H-CDR1, H-CDR2, and H-CDR3 comprising the amino acid sequences shown in SEQ ID NO. 44, SEQ ID NO. 42, and SEQ ID NO. 36, respectively; and L-CDR1, L-CDR2, and L-CDR3 comprising the amino acid sequences shown in SEQ ID NO. 37, SEQ ID NO. 38, and SEQ ID NO. 39, respectively.

4. The use according to any one of claims 1 to 3, wherein the heavy chain variable region (VH) and light chain variable region (VL) respectively comprise a combination of amino acid sequences selected from:
(1) the amino acid sequence shown in SEQ ID NO. 1, or an amino acid sequence having at least 75% identity with said amino acid sequence; and the amino acid sequence shown in SEQ ID NO. 2, or an amino acid sequence having at least 75% identity with said amino acid sequence;
(2) the amino acid sequence shown in SEQ ID NO. 3, or an amino acid sequence having at least 75% identity with said amino acid sequence; and the amino acid sequence shown in SEQ ID NO. 4, or an amino acid sequence having at least 75% identity with said amino acid sequence;
(3) the amino acid sequence shown in SEQ ID NO. 5, or an amino acid sequence having at least 75% identity with said amino acid sequence; and the amino acid sequence shown in SEQ ID NO. 6, or an amino acid sequence having at least 75% identity with said amino acid sequence;
(4) the amino acid sequence shown in SEQ ID NO. 7, or an amino acid sequence having at least 75% identity with said amino acid sequence; and the amino acid sequence shown in SEQ ID NO. 8, or an amino acid sequence having at least 75% identity with said amino acid sequence;
(5) the amino acid sequence shown in SEQ ID NO. 9, or an amino acid sequence having at least 75% identity with said amino acid sequence; and the amino acid sequence shown in SEQ ID NO. 10, or an amino acid sequence having at least 75% identity with said amino acid sequence; or
(6) the amino acid sequence shown in SEQ ID NO. 11, or an amino acid sequence having at least 75% identity with said amino acid sequence; and the amino acid sequence shown in SEQ ID NO. 12, or an amino acid sequence having at least 75% identity with said amino acid sequence.

5. The use according to any one of claims 1 to 4, wherein the anti-IL-11 antibody is a humanized antibody;
preferably, the anti-IL-11 antibody further comprises an IgG heavy chain constant region and/or a κ or λ light chain constant region; more preferably, the anti-IL-11 antibody is an IgG1 or κ monoclonal antibody.

6. The use according to any one of claims 1 to 5, wherein the anti-IL-11 antibody or antigen-binding fragment thereof comprises a heavy chain (HC) and light chain (LC) respectively comprising a combination of amino acid sequences selected from:
(1) the amino acid sequence shown in SEQ ID NO. 13, or an amino acid sequence having at least 75% identity with said amino acid sequence; and the amino acid sequence shown in SEQ ID NO. 14, or an amino acid sequence having at least 75% identity with said amino acid sequence;
(2) the amino acid sequence shown in SEQ ID NO. 15, or an amino acid sequence having at least 75% identity with said amino acid sequence; and the amino acid sequence shown in SEQ ID NO. 16, or an amino acid sequence having at least 75% identity with said amino acid sequence;
(3) the amino acid sequence shown in SEQ ID NO. 17, or an amino acid sequence having at least 75% identity with said amino acid sequence; and the amino acid sequence shown in SEQ ID NO. 18, or an amino acid sequence having at least 75% identity with said amino acid sequence;
(4) the amino acid sequence shown in SEQ ID NO. 19, or an amino acid sequence having at least 75% identity with said amino acid sequence; and the amino acid sequence shown in SEQ ID NO. 20, or an amino acid sequence having at least 75% identity with said amino acid sequence;
(5) the amino acid sequence shown in SEQ ID NO. 21, or an amino acid sequence having at least 75% identity with said amino acid sequence; and the amino acid sequence shown in SEQ ID NO. 22, or an amino acid sequence having at least 75% identity with said amino acid sequence; or
(6) the amino acid sequence shown in SEQ ID NO. 23, or an amino acid sequence having at least 75% identity with said amino acid sequence; and the amino acid sequence shown in SEQ ID NO. 24, or an amino acid sequence having at least 75% identity with said amino acid sequence.

7. The use according to any one of claims 1 to 6, wherein the tumor is a tumor associated with IL-11 expression, such as non-small cell lung cancer, pancreatic cancer, or cervical cancer.

8. The use according to any one of claims 1 to 7, wherein the drug is used for treating a mammal, preferably a human.

9. A method for treating a tumor, comprising administering to a subject in need thereof an anti-IL-11 antibody or antigen-binding fragment thereof.

10. The method according to claim 9, wherein the tumor is a tumor associated with IL-11 expression, such as non-small cell lung cancer, pancreatic cancer, or cervical cancer; the anti-IL-11 antibody or antigen-binding fragment thereof is as defined in any one of claims 3 to 6.
